# EUROPEAN PATENT SPECIFICATION

(11) **EP 1 386 971 B1**
(45) Date of publication and mention of the grant of the patent: **30.05.2012**
(21) Application number: 02718595.8
(22) Date of filing: 16.04.2002
(51) Int. Cl.: C12Q 1/42, G01N 21/78, G01N 33/50

(54) **METHOD OF ASSAYING BIOLOGICAL COMPONENT**
TESTVERFAHREN FÜR EINEN BIOLOGISCHEN BESTANDTEIL
METHODE D'ANALYSE DE CONSTITUANT BIOLOGIQUE

(30) Priority: 17.04.2001 JP 2001118180
(43) Date of publication of application: 04.02.2004
(73) Proprietor: SYSMEX CORPORATION, Chuo-ku Kobe-shi Hyogo 651-0073 (JP)
(72) Inventor: UEDA, Yoko, Kobe-shi, Hyogo 651-2241 (JP); YAMASHITA, Kazuaki, Kobe-shi, Hyogo 651-2241 (JP)
(74) Representative: HOFFMANN EITLE
(86) International application number: PCT/JP2002/003758
(87) International publication number: WO 2002/086151

(56) References cited:
- EP-A2- 0 882 800
- JP-A- 6 113 895
- JP-A- 11 046 794
- JP-A- 51 016 989
- JP-A- 2000 135 099
- PATENT ABSTRACTS OF JAPAN vol. 0061, no. 91 (C-127), 30 September 1982 (1982-09-30) & JP 57 102197 A (WAKO PURE CHEM IND LTD), 25 June 1982 (1982-06-25)
- PATENT ABSTRACTS OF JAPAN vol. 0121, no. 28 (C-489), 20 April 1988 (1988-04-20) & JP 62 248500 A (KANTO KAGAKU KK), 29 October 1987 (1987-10-29)

## Description

### [Field of the Invention]

The present invention relates to uses of a reagent for assaying a biological component in a field of clinical diagnosis that enable to avoid an interference of a coexisting non-objective substance on its assay result.

### [Prior Art]

At present, for assaying a serum component in a field of clinical chemistry, a method is generally prevailing in which, when an objective component is an enzyme, a compound as the substrate is reacted with the enzyme to produce product, which is then measured to calculate the enzymatic activity. When an objective component is an other substance than an enzyme, a method called "Enzyme Method" is generally prevailing in which the substance is reacted with an enzyme specific to the component to produce a product, which is then measured to calculate the amount of the objective component.

Among a variety of components in a body fluid, a reductive substance such as ascorbic acid, hemoglobin or bilirubin brings about an influence of a negative error based on reduction activity, and furthermore a coloring matter such as hemoglobin or bilirubin gives a positive or negative error depending on measurement wavelength. It is also generally known that light absorption of the coloring matter itself changes by the influence of light and ingredient(s) in an assay reagent composition with time passing during measurement to give an influence on the assay result. Such an influence is called interference.

In any assay method, various means have been studied for avoiding an interference brought about by a body liquid component. For examples, there are many reports on means only for avoiding the interference of hemoglobin or bilirubin. Known means for avoiding interference of hemoglobin include a means using thiourea, a means using a cationic surfactant and an amphoteric surfactant, a means using a cationic surfactant and an amphoteric surfactant and an anionic surfactant, a means using an alkylsulfonate, and a means using two lights each having different wavelength for measurement.

JP 6-113895 A and JP 51016989 A relate to methods and reagents for alkaline phosphatase (ALP) assay measurements. The reagents of JP 6-113895 A and JP 51016989 A contain thiodiglycolic acid as a stabilizer. However, they do not disclose a use of thiodiglycolic acid for avoiding an interference of hemolytic hemoglobin in a method for assaying an activity of alkaline phosphatase (ALP).

The above-mentioned means are means for avoiding interference in a method in which coloring is resulted mainly by combining an oxidase with a peroxidase. In addition, some of them employ a substrate decomposition coloring method such as the leucine aminopeptidase assaying method to avoid the interference. However, the problem in each interference brought about by hemoglobin produced by hemolysis is not satisfactorily solved yet.

### [Description of the Invention]

An object of the present invention is to provide uses of a reagent for assaying a biological component that enable to avoid an interference of a non-objective substance coexisting in an assay sample, such as hemoglobin produced by hemolysis, on the assay result.

The present inventors have made a diligent study and found out, to accomplish the invention, that the addition of at least thiodiglycolic acid in a reagent can avoid the interference of hemolytic hemoglobin.

Namely, the invention is as follows: A use according to claims 1 or 2.

### [Brief Description of Drawings]

Fig.1 shows the effect of added thiodiglycolic acid on the interference of conjugated bilirubin in the ALP assay system in comparison with no addition (Reference Example 1).
Fig.2 shows the effect of added thiodiglycolic acid on the interference of hemolytic hemoglobin in the ALP assay system in comparison with no addition (Reference Example 1).
Fig.3 shows the effect of added SDS on the interference of hemolytic hemoglobin in the ALP assay system in comparison with no addition (Reference Example 1).

### [Best Mode of the Invention]

A biological component to be assayed in the invention may be any component contained in a living body, and is not limited to a specific one. The invention can be applied to an assaying of a component contained in a body fluid such as blood, serum, plasma, urine, stool(feces), semen, cerebrospinal fluid, saliva, sweat, tear, ascites and amniotic fluid, or in an extract liquid from an organ, a tissue or a cell, of the human or animals. Example of such component includes a substance that can be measured by a spectrophotometer using absorbance or reflectance at a specific wavelength of light as the index. For example, a component having an enzyme activity by itself and an enzyme-labeled component for assaying the enzyme activity are included. More specifically, an alkaline phosphatase (ALP) activity in a living body or an ALP isozyme activity can be used as an index for assaying a biological component.

A reagent composition prepared by simply separating a conventional ALP assay reagent into a two-reagent system may give influence on the assay result of ALP activity caused by each interference of hemolytic hemoglobin. A means for avoiding these interferences in the ALP assaying system has been studied and it has been found out that the addition of at least thiodiglycolic acid exhibits some effects on avoiding the interference of hemolytic hemoglobin. The example includes a sulfur-containing oxoacid, a thioacid and their salt, ammonium sulfite, calcium sulfite, sodium sulfite, ammonium thiosulfate, sodium thiosulfate, potassium thiosulfate, thiodiglycolic acid, methionine and thiourea. More preferably, at least thiodiglycolic acid is added in a reagent to exhibit the effect. SDS, which has an action for avoiding the interference of hemolytic hemoglobin, can be used in combination with a compound selected from the above. Thus, the invention has been completed based on the finding that SDS can be used in combination to enable to avoid the interference of hemolytic hemoglobin.

The invention will be described in detail with reference to ALP below, but the assay object of the invention is not limited to ALP.

Human serum ALP is a membrane-bound enzyme that localizes exclusively outside a biomembrane and decomposes many phosphate compounds prevailing in organs in a body. The human serum ALP is a glycoprotein formed by coupling a sugar chain to a dimmer structure of two subunit molecules. The ALP activity is found to increase, for examples, in a disease such as rachitis, fibrous osteitis, hyperparathyroidism, Behcet's syndrome, metastatic bone cancer, osteomalacia, obstructive jaundice, acute hepatitis, cirrhosis, or metastatic hepatoma.

The assay of a biological component according to the invention can be employed for assaying ALP contained in a body fluid such as blood, serum, plasma, urine, stool, semen, cerebrospinal fluid, saliva, sweat, tear, ascites and amniotic fluid, or in an extract liquid from an organ, a tissue or a cell, of the human or animals. The assaying method also can be employed for assaying the activity of an ALP isozyme.

In the invention, the coloring compound dissociated by the action of ALP is a substance dissociated by the action of ALP from an ALP assaying substrate. The ALP assaying substrate includes, but is not limited to, glycerophosphoric acid, p-nitrophenyl phosphoric acid and phenyl phosphoric acid. Generally, p-nitrophenyl phosphoric acid is used. The coloring compound dissociated from each ALP assaying substrate is p-nitrophenol.

The assaying of ALP activity of the invention using the above substrate can determine the activity by measuring the decomposition velocity of the substrate with time passing followed by calculation from the increase of the absorbance, or by employing the endpoint method. Additionally, in the assaying of ALP activity using p-nitrophenyl phosphoric acid as the substrate, a method using a buffer such as a diethanolamine (DEA) buffer, a 2-(ethylamino) ethanol (EAE) buffer, an N-methyl-D-glucamine (MEG) buffer, or a 2-amino-2-methyl-1-propanol (AMP) buffer [See: Kensa to Gijutu, vol.29, No. 3, p.225-230 (2001)] is known. The uses of the assay reagent of the invention exhibit the effect particularly when the AMP is used.

In the invention, thiodiglycolic acid is added to the assay reagent, in order to avoid each interference of hemolytic hemoglobin, to a final concentration of 0.5-100mM, preferably 1.0-50mM, and more preferably 2.0-30mM. Here, the final concentration means a concentration relative to the final amount of the solution prepared to make an assay sample and the assay reagent react in the assaying system.

In addition to thiodiglycolic acid, SDS is also contained in the ALP assay reagent of the invention to avoid the interference of hemolytic hemoglobin. A final concentration of SDS is 0.001-1.0%(W/V), preferably 0.005-0.5%(W/V), and more preferably 0.01-0.1% (W/V).

The invention encompasses a use of an ALP assay reagent comprising thiodiglycolic acid and further additional SDS.

### Examples

The invention will be described below by examples, however it is not limited to them.

### Reference Example 1

In an assaying system of the samples containing various concentrations of conjugated bilirubin or hemolytic hemoglobin, an effect of adding thiodiglycolic acid or SDS to a reagent was studied.

Assay reagents were prepared as follows:

| (First reagent) | | |
|---|---|---|
| | 2-amino-2-methyl-1-propanol (AMP) | 350mM |
| | Zinc sulfate | 1.36mM |
| | Magnesium acetate | 2.71mM |
| | N-hydroxyethylethylenediamine triacetate (HEDTA) pH 10.4 (30°C) | 2.71mM |

| (Second reagent) | | |
|---|---|---|
| | 2-amino-2-methyl-1-propanol (AMP) | 350mM |
| | 4-nitrophenyl phosphoric acid pH 10.4 (30°C) | 65.1mM |

Two kinds of reagent were prepared by adding 30mM of thiodiglycolic acid or SDS to be at 0.5%, to the first reagent.

Assay samples were prepared according to a following manner.

### (a) Serum samples having different concentrations of conjugated bilirubin

One volume of a conjugated bilirubin-free aqueous solution or each of conjugated bilirubin (for INTERFERENCE CHECK; by INTERNATIONAL REAGENTS CORPORATION)-containing aqueous solutions arranged was mixed with nine volumes of control serum QAP trol IX (by INTERNATIONAL REAGENTS CORPORATION) to prepare a dilution series of the serum having a maximum conjugated bilirubin concentration of 22mg/dL as assay samples.

### (b) Serum samples having different concentrations of hemoglobin

One volume of a hemolytic hemoglobin-free aqueous solution or each hemolytic hemoglobin(for INTERFERENCE CHECK; by INTERNATIONAL REAGENTS CORPORATION)-containing aqueous solutions was mixed with nine volumes of control serum QAP trol IX (by INTERNATIONAL REAGENTS CORPORATION) to prepare a dilution series of the serum having a maximum hemoglobin produced by hemolysis concentration of 480mg/dL as assay samples.

Measurement was carried out under the following conditions.

210µL of the first reagent was added to 5µL of an assay sample, followed by preheating at 37°C for 5min, and 70µL of the second reagent was added. After stirring, increase speed of absorbance per unit time was compared at 405nm (primary wavelength) and 505nm (secondary wavelength). Then, based on the absorbance increase speed of an enzyme solution with the known ALP activity value, the enzyme activity of the respective assay samples was determined.

HITACHI Automatic Analyzer model 7170 was used for measurement.

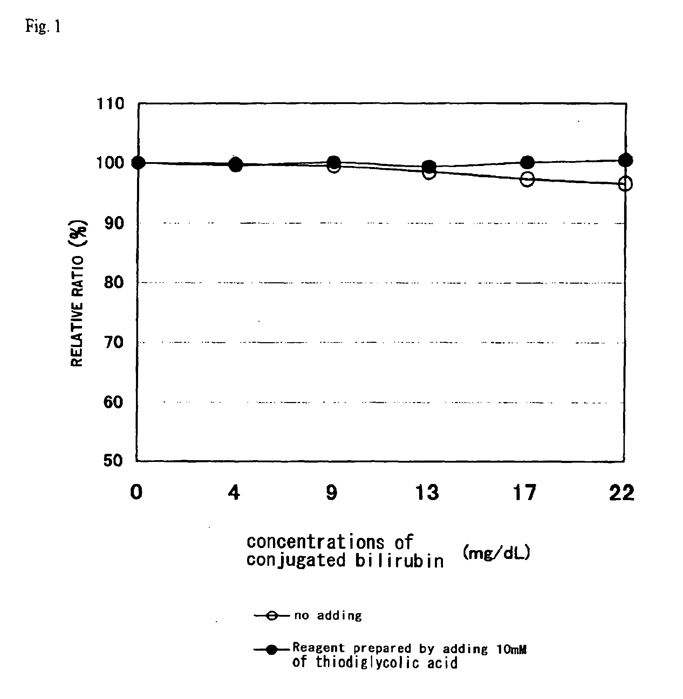

Results are shown in Figs. 1-3.
Fig. 1 reveals that the addition of thiodiglycolic acid can avoid the interference of respective concentrations of conjugated bilirubin.

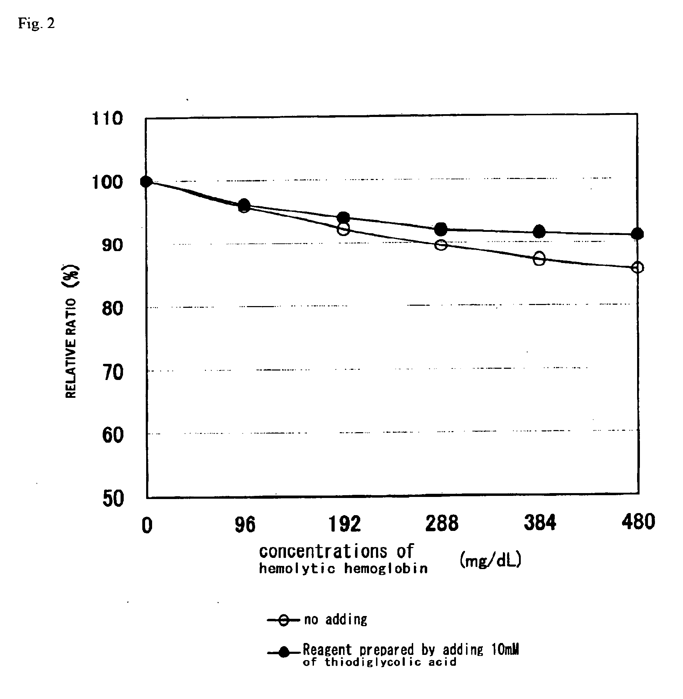

Fig. 2 reveals that the addition of thiodiglycolic acid can reduce the interference of respective concentrations of hemoglobin produced by hemolysis.

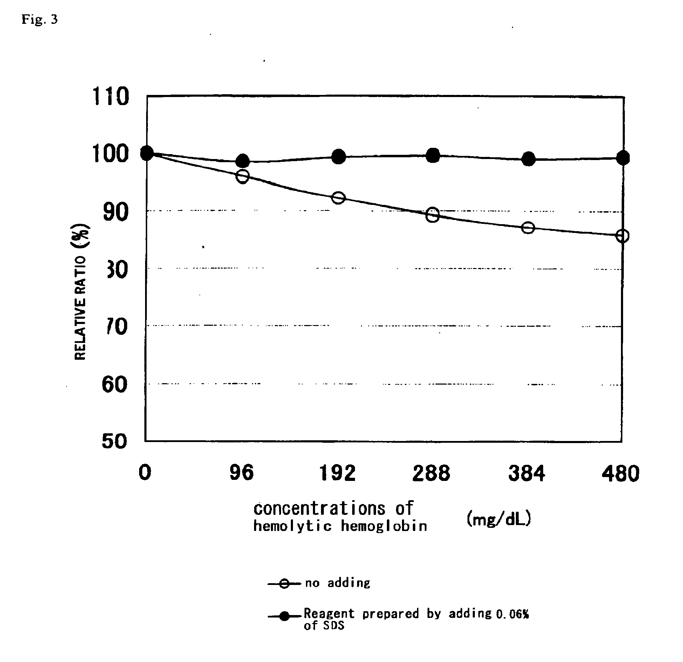

Fig. 3 makes sure that the addition of SDS can avoid the interference of respective concentrations of hemoglobin produced by hemolysis.

### Example 2

For assay samples containing conjugated bilirubin or hemolytic hemoglobin (hemolyzedHb), the effect of adding thiodiglycolic acid and SDS in the assay reagent was studied. Assay samples and assay reagents were prepared as follows. Measurement was performed same as Reference Example 1.

### (Preparation of assay sample)

Each assay sample was prepared as in Reference Example 1 to have a conjugated bilirubin concentration of 21.6mg/dL or a hemolyzed Hb concentration of 480mg/dL.

### (Preparation of reagent)

The first reagent and the second reagent were prepared as in Reference Example 1. Separately, a combination of thiodiglycolic acid and SDS was added to the first reagent to prepare a reagent containing thiodiglycolic acid and SDS at 30mM and 0.5%(W/V) respectively.

The effect of the added thiodiglycolic acid and SDS was evaluated based on a value relative to the measured value of the assay sample without conjugated bilirubin and hemolyzed Hb, which being defined as 100%.

The result is shown in Table 1. Table 1 reveals that the addition of thiodiglycolic acid and SDS can avoid the interference of conjugated bilirubin and hemolytic hemoglobin (Hemolytic Hb).

**(Table 1)**

| Assay sample | No addition | (%) addition |
|---|---|---|
| Conjugated Bilirubin (21.6mg/dL) added | 97 | 100 |
| Hemolyzed Hb (480mg/dL) added | 84 | 100 |

| | | |
|---|---|---|
| (No addition: the reagent system without thiodiglycolic acid and SDS) (Addition: the reagent system with thiodiglycolic acid and SDS) | | |

### [Industrial applicability]

As described above, the uses of the reagent for assaying a biological component according to the invention enable to avoid the interference of hemolytic hemoglobin to obtain an exact assay value of the biological component.

## Claims

1. Use of a reagent comprising thiodiglycolic acid and sodium lauryl sulfate for avoiding the interference of hemolytic haemoglobin in a method for assaying an activity of alkaline phosphatase (ALP).

2. Use of thiodiglycolic acid and sodium lauryl sulfate for avoiding the interference of hemolytic hemoglobin in a method for assaying an activity of alkaline phosphatase (ALP).

## Patentansprüche

1. Verwendung eines Reagens, das Thiodiglykolsäure und Natriumlaurylsulfat umfasst, zur Vermeidung der Interferenz von hämolytischem Hämoglobin in einem Verfahren zum Testen einer Aktivität der alkalischen Phosphatase (ALP).

2. Verwendung von Thiodiglykolsäure und Natriumlaurylsulfat zur Vermeidung der Interferenz von hämolytischem Hämoglobin in einem Verfahren zum Testen einer Aktivität der alkalischen Phosphatase (ALP).

## Revendications

1. Utilisation d'un réactif comprenant de l'acide thiodiglycolique et du laurylsulfate de sodium pour éviter l'interférence de l'hémoglobine hémolytique dans un procédé pour tester une activité de phosphatase alcaline (ALP).

2. Utilisation de l'acide thiodiglycolique et du laurylsulfate de sodium pour éviter l'interférence de l'hémoglobine hémolytique dans un procédé pour tester une activité de phosphatase alcaline (ALP).
